# EUROPÄISCHE PATENTSCHRIFT

(11) **EP 2 638 001 B1**
(45) Veröffentlichungstag und Bekanntmachung des Hinweises auf die Patenterteilung: **02.07.2014**
(21) Anmeldenummer: 11778923.0
(22) Anmeldetag: 07.11.2011
(51) Int. Cl.: C07C 209/08, C07C 209/62, C07C 211/24

(54) **VERFAHREN ZUR HERSTELLUNG VON 1,2-DIFLUORETHYLAMIN AUSGEHEND VON PROP-2-EN-1-AMIN**
METHOD FOR PRODUCING 2,2-DIFLUOROETHYLAMINE BASED ON PROP-2-EN-1-AMINE
PROCÉDÉ DE FABRICATION DE 2,2-DIFLUORÉTHYLAMIN À PARTIR DE LA PROP-2-ÈNE-1-AMINE

(30) Priorität: 12.11.2010 US 413059 P; 12.11.2010 EP 10191059
(43) Veröffentlichungstag der Anmeldung: 18.09.2013
(73) Patentinhaber: Bayer Intellectual Property GmbH, 40789 Monheim am Rhein (DE)
(72) Erfinder: LUI, Norbert, 51519 Odenthal (DE); FUNKE, Christian, 42799 Leichlingen (DE); HEINRICH, Jens-Dietmar, 51381 Leverkusen (DE); MÜLLER, Thomas, Norbert, 40789 Monheim (DE)
(74) Vertreter: BIP Patents
(86) Internationale Anmeldenummer: PCT/EP2011/069546
(87) Internationale Veröffentlichungsnummer: WO 2012/062703

(56) Entgegenhaltungen:
- WO-A1-2009/036901
- US-A- 4 030 994
- US-A- 5 773 617
- OBWOHLM. HUDLICKY: "Chemistry of Organofluorine Compounds", 1976, XP009146465, Seiten 489-490, das ganze Dokument
- GUIDO VERNIEST ET AL: "Synthesis and Reactivity of 1-Substituted 2-Fluoro- and 2,2-Difluoroaziridines", J. ORG. CHEM., Bd. 72, Nr. 22, 2007, Seiten 8569-8572, XP002630131,

## Beschreibung

Die vorliegende Erfindung betrifft ein Verfahren zur Herstellung von 2,2-Difluorethylamin durch Umsetzung von Prop-2-en-1-amin mit 2,2-Difluor-1-halogenethan.

2,2-Difluorethylamin ist eine wichtige Zwischenverbindung bei der Wirkstoffherstellung. Es sind verschiedene Herstellungsmethoden für 2,2-Difluorethylamin bekannt.

Donetti et al. (J. Med. Chem. 1989, 32, 957-961) beschreiben die Synthese von 2,2-Difluorethylamin-Hydrochlorid ausgehend von 2,2-Difluoracetamid. Hierbei wird mit einer Diboran-Lösung in Tetrahydrofuran (THF) das gewünschte Amin hergestellt. Die Ausbeute beträgt 48%.

Kluger et al. (JACS 1982, 104, 10, 2891-2897) beschreiben die Synthese von 2,2-Difluorethylamin ausgehend vom Amid mit Natriumboranat und Bortrifluoridetherat. Die Ausbeute beträgt 60%. Vyazkov, V.A. et al. (Vyazkov, V. A.; Gontar, A. F.; Grinevskaya, V. K.; Igoumnova, E. V.; Igoumnov, S. M. A.)N. Nesmeyanov Institute of Organoelement Compounds, Russian Academy of Sciences, Moscow, Russia Fluorine Notes (2009), 65) beschreiben ebenfalls die Reduktion mit Natriumboranat in einer Ausbeute von 50 - 65 %.

Auch Kollonitsch (US 4,030,994) beschreibt eine Synthese von 2,2-Difluorethylamin, nämlich die Umsetzung von Ethylamin mit Fluoroxytrifluormethan in Fluorwasserstoff unter UV Bestrahlung.

Swarts beschreibt in seinem Artikel mit dem Titel "Über einige fluorhaltige Alkylamine" (Chem. Zentralblatt, Band 75, 1904, Seiten 944-945) die Herstellung von 2,2-Difluorethylamin und von Tetrafluorethylamin, mit anschließender Abtrennung der beiden Produkte durch fraktionierte Destillation oder als Chlorhydrat- oder Oxalat-Salze nach vorheriger Umwandlung der erhaltenen Produkte. Swarts verwendet als Ausgangsverbindung 1-Brom-2,2-difluorethan und erhitzt dieses über einen verhältnismäßig langen Zeitraum, nämlich 3 Tage, im Reaktionsrohr mit 2 Mol alkoholischem Ammoniak auf relativ hohe Temperaturen, nämlich 125 - 145 °C. Die Ausgangsverbindung wird vollständig in die Verbindungen Difluorethylamin und Tetrafluorethylamin umgesetzt.

Die Herstellung von 2,2-Difluorethylamin wird auch bei Dickey et al. (Industrial and Engineering Chemistry 1956, Nr. 2, 209-213) beschrieben. 2,2-Difluor-1-chlorethan wird dort mit 2 8 %-igem Ammoniumhydroxid, d.h. 28 %-iger wässriger Ammoniak-Lösung, in einem Autoklaven (rocking autoclave) zur Reaktion gebracht. Die Reaktionsmischung wird für 31 Stunden auf Temperaturen von 135° bis 140 °C erhitzt. Nach Beendigung der Reaktion wird die Reaktionsmischung filtriert und das Amin vom Reaktionsgemisch abdestilliert. Da sich aber noch eine Menge Ammoniak und etwas Wasser im Destillat befindet, wird das Amin über Natriumhydroxid getrocknet und nochmals destilliert. Das Amin wurde so in einer Ausbeute von 65 % erhalten.

Dieses Verfahren ist nachteilig, denn es benötigt - genauso wie das Verfahren nach Swarts - eine sehr lange Reaktionszeit von 31 Stunden und die Ausbeute von 65 % ist eher gering. Gleichzeitig ist die Reaktionsmischung stark korrodierend, da der wässrige Ammoniak in Kombination mit den im Reaktionsgemisch vorhandenen Chlorid- und Fluoridionen bei den im Verfahren verwendeten hohen Temperaturen metallische Werkstoffe angreift.

Alle diese bekannten Verfahren sind nachteilig, insbesondere weil sie sich nicht im wirtschaftlich sinnvollen großtechnischen (industriellen) Maßstab durchführen lassen. Die geringe Ausbeute und der Einsatz von teuren und gefährlichen Chemikalien wie z.B. Natriumboranat/BF₃ oder Diboran verhindern, dass die Verfahren nach Donetti et al. und Kluger et al. für die großtechnische Herstellung von 2,2-Difluorethylamin geeignet sind. Das Verfahren nach Kollonitsch et al. verwendet gefährliche Chemikalien und es wird kein reines 2,2-Difluorethylamin erhalten. Das Verfahren nach Dickey et al. und das Verfahren nach Swarts sind ebenfalls für den großtechnischen Einsatz ungeeignet bzw. unwirtschaftlich, denn sie benötigen sehr lange Reaktionszeiten und sind gleichzeitig nicht selektiv, so dass die Ausbeuten der Verfahren unbefriedigend sind.

Weiterhin ist der Einsatz von Ammoniak bei hohen Temperaturen problematisch, da man spezielle druckfeste Apparaturen benötigt, was sicherheitstechnisch anspruchsvoll und teuer ist.

Ausgehend von den bekannten Verfahren zur Herstellung von 2,2-Difluorethylamin stellt sich nun die Aufgabe, wie 2,2-Difluorethylamin einfach und kostengünstig hergestellt werden kann. Unter kostengünstigen Verfahren werden solche Verfahren verstanden, die ohne großen finanziellen Aufwand durchzuführen sind, weil die Ausgangsstoffe beispielsweise ungefährlich sind, keine sonstigen technischen Probleme auftauchen, beispielsweise weil das Reaktionsgemisch korrodierend wirkt, und/oder das gewünschte 2,2-Difluorethylamin in einer ausreichend hohen Ausbeute und Reinheit erhalten wird, weil etwa die Reaktion weitgehend selektiv verläuft.

Es wurde nun ein besonders vorteilhaftes Verfahren zur Herstellung von 2,2-Difluorethylamin gefunden, mit dem die oben genannten Nachteile vermieden werden und das einfach im großtechnischen Maßstab einzusetzen ist. Im erfindungsgemäßen Verfahren wird in einem ersten Schritt eine 2,2-Difluor-1-halogenethan-Verbindung unter vergleichsweise milden Reaktionsbedingungen und vergleichsweise kurzer Reaktionszeit selektiv zum gewünschten N-(2,2-Difluorethyl)prop-2-en-1-amin umgesetzt. In einem zweiten Schritt wird die Allylgruppe mit Hilfe eines Katalysators wieder entfernt und das gewünschte 2,2-Difluorethylamin entsprechend erhalten.

Gegenstand der Erfindung ist somit ein Verfahren zur Herstellung von 2,2-Difluorethylamin der Formel (I)

CHF₂CH₂NH₂ (I)

das die folgenden Schritte (i) und (ii) umfasst:

Schritt (i) - Alkylierung: Umsetzung von 2,2-Difluor-1-halogenethan der Formel (II)

CHF₂₋CH₂Hal (II),

worin Hal für Chlor, Brom oder Jod steht, Hal steht bevorzugt für Chlor oder Brom, ganz bevorzugt für Chlor,
mit Prop-2-en-1-amin der Formel (III) 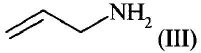 zu N-(2,2-Difluorethyl)prop-2-en-1-amin der Formel (IV) 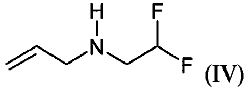 vorzugsweise in Gegenwart eines Säurefängers
und

Schritt (ii): Entfernung der Allylgruppe (Deallylierung) aus dem in Schritt (i) erhaltenen N-(2,2-Difluorethyl)prop-2-en-1-amin der Formel (IV), wodurch 2,2-Difluorethylamin der Formel (I) oder ein Salz davon erhalten wird, vorzugsweise in Gegenwart eines Katalysators und gegebenenfalls in Gegenwart eines Nucleophils.

Das erfindungsgemäße Verfahren kann durch das folgende Schema veranschaulicht werden:

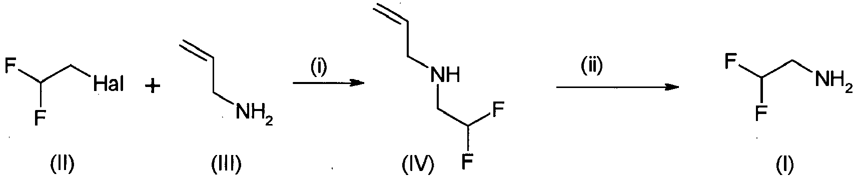

Das gewünschte 2,2-Difluorethylamin wird mit dem erfindungsgemäßen Verfahren mit guten Ausbeuten, kurzen Reaktionszeiten und in hoher Reinheit erhalten, weshalb eine umfangreiche Aufarbeitung des unmittelbaren Reaktionsprodukts im Allgemeinen nicht erforderlich ist.

Gegenstand der Erfindung ist gleichfalls das Verfahren des Schritts (i) zur Herstellung von N-(2,2-Difluorethyl)prop-2-en-1-amin umfassend die Umsetzung von 2,2-Difluor-1-chlorethan mit Prop-2-en-1-amin in Gegenwart eines Säurefängers und gegebenenfalls in Gegenwart eines Katalysators, das die für Schritt (i) beschriebenen Verfahrensschritte, Reaktionsbedingungen und Reaktionspartner beinhaltet.

Gegenstand der Erfindung ist weiterhin die Verwendung von N-(2,2-Difluorethyl)prop-2-en-1-amin zur Herstellung von 2,2-Difluorethylamin, die die für Schritt (ii) beschriebenen Verfahrensschritte, Reaktionsbedingungen und Reaktionspartner beinhaltet.

Obwohl aus M. Hudlicky in "Chemistry of Organofluorine Compounds", 2. Auflage, 1976, S. 489-490 und Houben Weyl, E 10b/2, S. 92-98 bekannt ist, dass 2,2-Difluor-1-halogenethan unter basischen Bedingungen unter Abspaltung von HHal (HCl, HBr oder HJ) zu Vinylidenfluorid reagiert und es dadurch für die Umsetzung in Schritt (i) nicht mehr zur Verfügung steht, und obwohl aus J. Org. Chem. 2007, 72 (22) S. 8569 bekannt ist, dass 2,2-Difluorethylamine sehr reaktiv sind und es sehr wahrscheinlich ist, dass das erhaltene N-(2,2-Difluorethyl)prop-2-en-1-amin der Formel (IV) unter den Reaktionsbedingungen im Schritt (i) weiterreagiert, haben die Erfinder überraschenderweise gefunden, dass durch Schritt (i) des erfindungsgemäßen Verfahrens N-(2,2-Difluorethyl)prop-2-en-1-amin der Formel (IV) in guter Ausbeute und Reinheit erhalten wird, so dass eine umfangreiche Aufreinigung entfallen kann. Letztendlich wird deshalb auch die Zielverbindung 2,2-Difluorethylamin in einer sehr guter Ausbeute, bezogen auf die im Schritt (i) eingesetzten Edukte, erhalten.

Im Hinblick auf die Alkylierung im Schritt (i) haben die Erfinder - entgegen der Erwartung, dass vermehrt Doppel- oder Mehrfachalkylierungen auftreten - gefunden, dass wenn die Summe der molaren Mengen von umzusetzendem Prop-2-en-1-amin der Formel (III) (Allylamin) und Säurefänger kleiner ist als die molare Menge an eingesetzten 2,2-Difluorhalogenethan der Formel (II), sehr hohe Ausbeuten erzielt werden. Wird Allylamin sowohl als Edukt als auch als Säurefänger eingesetzt, so gilt auch hier, dass die Summe der molaren Menge an Allylamin die umgesetzt wird und der molaren Menge an Allylamin, die als Säurefänger dient, geringer ist als die molare Menge an eingesetztem 2,2-Difluorhalogenethan der Formel (II).

Im erfindungsgemäßen Verfahren werden bevorzugt 2,2-Difluor-1-halogenethan-Verbindungen der Formel (II) eingesetzt, in denen Hal für Chlor oder Brom steht. Besonders bevorzugt wird die Verbindung 2,2-Difluor-1-chlorethan (CHF₂-CH₂Cl) eingesetzt.

Prop-2-en-1-amin der Formel (III) (Allylamin) ist bekannt und käuflich erhältlich.

Sofern nichts anderes angegeben, bezieht sich die Bezeichnung "Alkyl" - in Alleinstellung oder in Kombination mit anderen Begriffen, wie beispielsweise Alkoxy - auf lineare oder verzweigte gesättigte Kohlenwasserstoffketten mit bis zu 12 Kohlenstoffatomen, d.h. C₁-C₁₂-Alkyl, bevorzugt mit bis zu 6 Kohlenstoffen, d.h. C₁-C₆-Alkyl, besonders bevorzugt mit bis zu 4 Kohlenstoffen, d.h. C₁-C₄-Alkyl. Beispiele solcher Alkyle sind Methyl, Ethyl, n-Propyl oder Isopropyl, n-, i-, s- oder t-Butyl, n-Pentyl, n-Hexyl, n-Heptyl, n-Octyl, n-Nonyl, n-Decyl, n-Undecyl und n-Dodecyl. Die Alkyle können mit einem geeignetem Substituenten substituiert sein, z.B. mit Halogen.

Sofern nichts anderes angegeben steht "Halogen" oder "Hal" für Fluor, Chlor, Brom oder Jod.

Die Umsetzung von 2,2-Difluor-1-halogenethan der Formel (II) mit Prop-2-en-1-amin aus Schritt (i) kann in Substanz, d.h. ohne Hinzufügen eines Lösungsmittels oder in Gegenwart eines Lösungsmittels durchgeführt werden.

Im Falle dass in Schritt (i) ein Lösungsmittel zum Reaktionsgemisch hinzugefügt wird, wird es vorzugsweise in einer solchen Menge eingesetzt, dass das Reaktionsgemisch während des ganzen Verfahrens gut rührbar bleibt. Vorteilhafterweise wird, bezogen auf das Volumen des eingesetzten 2,2-Difluor-1-halogenethan, die 1- bis 50-fache Lösungsmittelmenge, bevorzugt die 2- bis 40-fache Lösungsmittelmenge, besonders bevorzugt die 2- bis 20-fache Lösungsmittelmenge verwendet. Unter Lösungsmittel werden erfindungsgemäß auch Gemische reiner Lösungsmittel verstanden. Geeignete Lösungsmittel sind alle unter den Reaktionsbedingungen inerten organischen Lösungsmittel. Erfindungsgemäß geeignete Lösungsmittel sind insbesondere Wasser, Ether (z.B. Ethylpropylether, Methyl-tert-butylether, n-Butylether, Anisol, Phenetol, Cyclohexylmethylether, Dimethylether, Diethylether, Dimethylglycol, Diphenylether, Dipropylether, Diisopropylether, Di-n-butylether, Diisobutylether, Diisoamylether, Ethylenglycoldimethylether, Isopropylethylether, Diethylenglykoldimethylether, Triethylenglykoldimethylether, Tetrahydrofuran, 2-Methyltetrahydrofuran, Dioxan und Polyether des Ethylenoxids und/oder Propylenoxids); Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe (z.B. Pentan, Hexan, Heptan, Oktan, Nonan wie die sogenannten "White Spirits" mit Komponenten mit Siedepunkten im Bereich beispielsweise von 40°C bis 250°C, Cymol, Benzinfraktionen innerhalb eines Siedeintervalls von 70°C bis 190°C, Cyclohexan, Methylcyclohexan, Petrolether, Ligroin, Benzol, Toluol, Xylol); halogenierte Aromate (z.B. Chlorbenzol, Dichlorbenzol); Amide (z.B. Hexamethylphosphorsäuretriamid, Formamid, N,N-Dimethyl-acetamid, *N*-Methyl-formamid, *N,N*-Dimethyl-formamid, *N,N*-Dipropyl-formamid, *N,N*-Dibutyl-formamid, *N*-Methyl-pyrrolidin, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N-*Formyl-piperidin, *N,N*'-1,4-Diformyl-piperazin); Nitrile (z.B. Acetonitril, Propionitril, n- oder iso-Butyronitril oder Benzonitril); Ketone (z.B. Aceton) oder Gemische davon.

Bevorzugte Lösungsmittel in Schritt (i) sind aromatische und/oder aliphatische Kohlenwasserstoffe, insbesondere Toluol, N,N-Dimethylacetamid, Tetramethylensulfoxid und *N*-Methyl-pyrrolidon.

Es ist erfindungsgemäß bevorzugt Schritt (i) in Substanz, d.h. ohne Lösungsmittel durchzuführen. Hierdurch kann das Verfahren noch kostengünstiger durchgeführt werden, weil die Lösungsmittel weder gekauft, noch nach Reaktion entsorgt werden müssen.

Die Umsetzung des Schritts (i) erfolgt vorteilhafterweise in Gegenwart eines oder mehrerer Säurefänger, die in der Lage sind, die bei der Reaktion frei werdende Halogenwasserstoff-Verbindung (d.h. HCl, HBr, HI) zu binden. Säurefänger sind Verbindungen, die in der Lage sind, eine Säure zu deaktivieren (neutralisieren).

Geeignete Säurefänger sind alle organische und anorganische Basen, die in der Lage sind, die frei werdenden Halogenwasserstoff-Verbindungen zu binden. Beispiele für organische Basen sind tertiäre Stickstoffbasen, wie z.B. tertiäre Amine, substituierte oder unsubstituierte Pyridine und substituierte oder unsubstituierte Chinoline, Triethylamin, Trimethylamin, N,N-Diisopropylethylamin, Tri-n-propylamin, Tri-n-butylamin, Tri-n-hexylamin, Tricyclohexylamin, N-Methyl-cyclohexylamin, N-Methylpyrrolidin, N-Methylpiperidin, N-Ethylpiperidin, N,N-Dimethylanilin, N-Methylmorpholin, Pyridin, 2-, 3-, 4-Picolin, 2-Methyl-5-ethyl-pyridin, 2,6-Lutidin, 2,4,6-Collidin, 4-Dimethylaminopyridin, Chinolin, Chinaldin, N,N,N,N-Tetramethylethylend i a m in, N , N-Dimethyl-l,4-diazacyclohexan, N,N-Di-ethyl-1,4-diazacyclohexan, 1,8-Bis-(Dimethylamino)naphthalin, Diazabicyclooctan (DABCO), Diazabicyclononan (DBN), Diazabicycloundecan (DBU), Butylimidazol und Methylimidazol.

Beispiele für anorganische Basen sind Alkali- oder Erdalkalimetallhydroxyde, Hydrogencarbonate oder Carbonate und sonstige anorganische wässrige Basen; bevorzugt sind z.B. Natriumhydroxid, Kaliumhydroxid, Lithiumhydroxid, Calciumhydroxid, Natriumcarbonat, Kaliumcarbonat, Natriumhydrogencarbonat, Kaliumhydrogencarbonat und Natriumacetat.

Das molare Verhältnis von Säurefänger, insbesondere von oben genannten Basen, zum eingesetzten Prop-2-en-1-amin liegt im Bereich von etwa 0,1 bis etwa 3, bevorzugt im Bereich von etwa 0,5 bis etwa 3, besonders bevorzugt im Bereich von etwa 0,7 bis etwa 1,3. Der Einsatz größerer Mengen an Base ist technisch möglich, führt jedoch zu einem Ausbeuteverlust.

Das molare Verhältnis von 2,2-Difluor-1-halogenethan zum eingesetzten Prop-2-en-1-amin liegt normalerweise im Bereich von etwa 30 : 1 bis etwa 1 : 3, bevorzugt im Bereich von etwa 10 : 1 bis etwa 1 : 2, besonders bevorzugt im Bereich von etwa 8 : 1 bis etwa 1 : 1.

In einer bevorzugten Ausführungsform dient das Prop-2-en-1-amin als Säurefänger, so dass kein weiterer Säurefänger zugesetzt werden muss. In diesem Fall liegt das molare Verhältnis von 2,2-Difluor-1-halogenethan zum eingesetzten Prop-2-en-1-amin normalerweise im Bereich von etwa 15 : 1 bis etwa 1 : 3, bevorzugt im Bereich von etwa 8 : 1 bis etwa 1 : 2,5, besonders bevorzugt im Bereich von etwa 4 : 1 bis etwa 1 : 2.

Das Prop-2-en-1-amin und die Base können zu dem 2,2-Difluor-1-halogenethan der Formel (II) auch zudosiert werden.

Obwohl Schritt (i) des erfindungsgemäßen Verfahrens im Allgemeinen ohne Zusatz eines Katalysators durchgeführt wird, können im Schritt (i) auch Katalysatoren, die die Umsetzung des Prop-2-en-1-amin mit 2,2-Difluorhalogenethan beschleunigen, verwendet werden. Durch Verwenden eines Katalysators wird die Reaktionstemperatur absenkt, wodurch auch der Eigendruck der Reaktionsmischung abgesenkt wird. Ist der Eigendruck nicht so hoch, kann unter technisch einfacheren Bedingungen gearbeitet werden.

Erfindungsgemäß geeignet sind insbesondere Alkalibromide und jodide (z.B. Natriumjodid, Kaliumjodid, Kaliumbromid); Ammoniumbromid und Ammoniumjodid; Tetraalkylammoniumbromide und -jodide (z.B. Tetraethylammoniumjodid); bestimmte Phosphoniumhalogenide, wie Tetraalkyl- oder Tetraarylphosphoniumhalogenide (z.B. Hexadecyltri-butyl-phosphoniumbromid, Stearyltributylphosphoniumbromid, Tetrabutylphosphoniumbromid, Tetraoctylphosphoniumbromid, Tetraphenylphosphoniumchlorid und Tetraphenylphosphoniumbromid), Tetrakis(dimethylamino)phosphoniumbromid, Tetrakis(diethylamino)phosphoniumbromid, Tetrakis(dipropylamino)phosphoniumchlorid und -bromid; sowie Bis(dimethylamino)[(1,3-dimethylimidazolidin-2-yliden)amino]methyliumbromid. Mischungen geeigneter Katalysatoren sind auch denkbar.

Von den vorgenannten, in Schritt (i) verwendbaren Katalysatoren sind Natriumjodid, Kaliumjodid, Kaliumbromid, Tetrabutylammoniumbromid und Tetraphenylphosphoniumbromid besonders geeignet, die Umsetzung des Schritts (i) zu beschleunigen. Natrium- und Kaliumjodid sind besonders hervorzuheben.

Der Katalysator kann auch *in-situ* erzeugt werden. Beispielsweise durch eine Reaktion von HBr oder HJ mit Ammoniak oder durch Zugabe von sehr reaktiven Alkylbromiden oder -jodiden (z.B. Methyl- oder Ethylbromid oder -jodid) erzeugt werden.

Falls im Schritt (i) ein Katalysator anwesend ist, so wird er, bezogen auf das eingesetzte 2,2-Difluor-1-halogenethan der Formel (II), in einer Konzentration von etwa 0,01 bis etwa 25 Gew.-% verwendet. Höhere Konzentrationen sind grundsätzlich möglich. Bevorzugt wird der Katalysator in einer Konzentration von etwa 0,2 bis etwa 25 Gew.-%, besonders bevorzugt von etwa 0,4 bis etwa 20 Gew.-%, ganz besonders bevorzugt von etwa 0,5 bis etwa 15 Gew.%. verwendet. Der Katalysator kann aber auch bevorzugt in einer Konzentration von etwa 0,05 bis etwa 3 Gew.%, von etwa 0,1 bis etwa 10 Gew.-% oder von etwa 0,5 bis etwa 10 Gew.-%. eingesetzt werden.

Die Umsetzung des Schrittes (i) wird grundsätzlich unter Eigendruck in einem druckstabilen geschlossenen Versuchsgefäß (Autoklav) durchgeführt. Der Druck während der Reaktion (d.h. der Eigendruck) ist abhängig von der verwendeten Reaktionstemperatur, dem verwendeten 2,2-Difluor-1-halogenethan, dem eingesetzten Katalysator und der Menge an Prop-2-en-1-amin. Gleichfalls hängt der Druck auch von dem verwendeten Lösungsmittel ab, falls Lösungsmittel im Schritt (i) anwesend ist. Ist eine Druckerhöhung gewünscht, so kann eine zusätzliche Druckerhöhung durch Zugabe eines Inertgases, wie Stickstoff oder Argon, erreicht werden.

Die Reaktionstemperatur in Schritt (i) kann in Abhängigkeit der verwendeten Ausgangsstoffe variieren. Wird im Schritt (i) kein Katalysator zugefügt, so wird Schritt (i) bei Innentemperaturen (d.h. der Temperatur, die im Reaktionsgefäß vorhanden ist) im Bereich von etwa 70°C bis etwa 200°C durchgeführt. Es ist bevorzugt, dass bei Durchführung des Reaktionsschrittes (i) die Innentemperatur im Bereich von etwa 90 °C bis etwa 150°C, besonders bevorzugt im Bereich von etwa 90°C bis etwa 140°C liegt. Es wurde festgestellt, dass wenn im bevorzugten Temperaturbereich gearbeitet wird, wenige Nebenreaktionen, insbesondere Mehrfachalkylierungen, auftreten.

Wird in Schritt (i) ein Katalysator verwendet, so erniedrigt sich die Reaktionstemperatur der Reaktionsmischung entsprechend. Dem Fachmann ist bekannt, inwieweit sich die Reaktionstemperatur bei Zufügen eines bestimmten Katalysators absenkt und er kann anhand von Routineversuchen oder anhand seines Fachwissens und anhand der vorgenannten Innentemperaturbereiche den für das spezielle Reaktionsgemisch optimalen Reaktions-Innentemperaturbereich finden.

Die Reaktionsdauer der Umsetzung in Schritt (i) liegt im Bereich von etwa 0,5 bis etwa 20 Stunden. Eine längere Reaktionsdauer ist möglich, jedoch wirtschaftlich nicht sinnvoll.

Die Aufarbeitung des Reaktionsgemisches aus Schritt (i) erfolgt entweder durch Filtration und anschließender fraktionierter Destillation oder durch Verwässerung (Zugabe von Wasser in dem gegebenenfalls Salze gelöst sind) der Reaktionsmischung, anschließender Phasentrennung und nachfolgender fraktionierter Destillation. Die Base oder das Prop-2-en-1-amin kann durch weiteren Base, z.B. Natronlauge, wieder freigesetzt und entsprechend in den Prozess wieder zurückgeführt werden.

Das in Schritt (i) hergestellte N-(2,2-Difluorethyl)prop-2-en-1-amin der Formel (IV) wird dann der Deallylierung des Schritts (ii) unterzogen, d.h. die Allylgruppe in N-(2,2-Difluorethyl)prop-2-en-1-amin der Formel (IV) wird entfernt (abgespalten).

Es ist bevorzugt, dass die Deallylierung in Gegenwart eines Katalysators stattfindet.

Methoden zur Spaltung einer allylischen C-N Bindung sind bekannt und beispielsweise in dem Übersichtssartikel von Escoubet, Stephanie; Gastaldi, Stephane; Bertrand, Michele in European Journal of Organic Chemistry (2005),(18), Seiten 3855-3873 beschrieben. In Bezug auf die Durchführung des Schritts (ii) wird auf diese Methoden hier vollumfänglich Bezug genommen. Die "Tsuji-Trost-Reaktion" ist gleichfalls eine Deallylierung. Sie ist die Palladium-katalysierte Allylierung von Nucleophilen wie C-aciden Verbindungen, Enolaten, Aminen und Phenolen mit Allylverbindungen wie Allylacetaten oder Allylbromiden.

Die Deallylierung kann durch Isomerisierung der Doppelbindung der Allylgruppe zu einem Enamin erfolgen, welches dann durch Hydrolyse gespalten werden kann (Reaktionsweg (2) in Schema 7) oder die Allylgruppe kann auf ein anionisches Nucleophil (Nu⁻) übertragen und das 2,2-Difluorethylamin freigesetzt werden (Reaktionswege (1) in Schema 7).

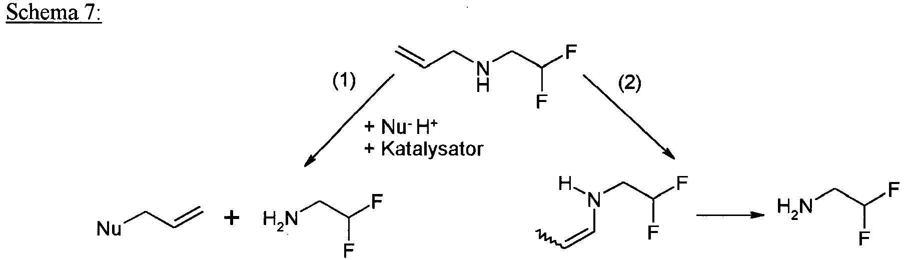

Erfolgt die Deallylierung wie in Schema 7 dargestellt nach Reaktionsweg (2) dann muss in Schritt (ii) eine Säure zur Spaltung des Enamins vorhanden sein. Beispiele solcher Säuren sind Methansulfonsäure, p-Toluolsulfonsäure, Ameisensäure und Essigsäure. Die Reaktionsbedingungen zur Abspaltung der Allylgruppe sind so zu wählen, dass das gebildete 2,2-Difluorethylamin stabil ist; insbesondere werden keine starken Basen zur Umlagerung verwendet, da es sonst zu Produktverlusten kommt. Starke Basen sind solche Basen, bei denen die Gleichgewichtsreaktion vollständig auf der Seite der OH⁻ Ionen liegen.

In einer bevorzugten Ausführungsform des Schritts (ii) findet die Entfernung der Allylgruppe aus N-(2,2-Difluorethyl)prop-2-en-1-amin in Gegenwart eines geeigneten Katalysators statt. Geeignete Katalysatoren sind heterogene oder homogene Katalysatoren, die ein oder mehrere Metalle der Gruppen 8 - 10 des Periodensystems enthalten. Die entsprechenden Katalysatoren können auch in geträgerter Form, beispielsweise auf Kohlenstoff (Kohle oder Aktivkohle), Aluminiumoxid, Bariumsulfat, Bariumcarbonat, Siliciumdioxid, Zirkondioxid, Calciumcarbonat oder Titandioxid aufgebracht, verwendet werden. Geeignete Metalle sind insbesondere Edelmetalle (z.B. Ruthenium, Palladium, Platin und Rhodium). Als homogene Katalysatoren eignen sich Palladium(II)chlorid, Palladium(II)acetat, Bis(acetylacetonat)palladium(II), Dichlorobis(triphenylphosphin)palladium(II), Tetrakis(triethylphosphin)palladium, Tetrakis(triphenylphosphin)palladium und Ruthenium(III)chlorid. Bevorzugt sind Palladium(0)-Katalysatoren, insbesondere Palladium-10%ig auf Kohle. Gleichfalls geeignet sind Palladium(II)chlorid, Palladium(II)acetat, Bis(acetylacetonat)palladium(II), Dichlorobis(triphenylphosphin)palladium(II), Tetrakis(triethylphosphin)palladium und Tetrakis(triphenylphosphin)palladium. Die Katalysatoren können sowohl in ihrer wasserfeuchten als auch trockenen Form eingesetzt werden.

Findet die Deallylierung des Schritts (ii) in Gegenwart eines Katalysators statt, dann wird der Katalysator, bezogen auf die eingesetzte Verbindung der Formel (IV), in einer Konzentration von etwa 0,001 bis etwa 20 Mol.-% verwendet. Bevorzugt wird der Katalysator in einer Konzentration von etwa 0,01 bis etwa 10 Mol.-% verwendet, besonders bevorzugt von etwa 0,01 bis etwa 5,0 Mol.-%.

Findet die Deallylierung des Schritts (ii) in Gegenwart eines Katalysators statt, dann ist es vorteilhaft, wenn eine Verbindung anwesend ist, die als Nucleophil fungiert. Typische Verbindungen, die als Nucleophile fungieren, und deshalb Nucleophile genannt werden, sind anionische Nucleophile wie Hydroxide, Alkoholate, Thiolate, Carbanionen, Halogenide, Peroxide, Cyanide und Azide. Die anionischen Nucleophile können in protonierter Form eingesetzt werden. Solche protonierten Nucleophile sind z.B. Thiole, Sulfinsäuren, 2-Mercaptobenzoesäure, Malonsäure und deren Derivate und β-Dicarbonylverbindungen, Barbitursäuren, wie z.B. N,N'-Dimethylbarbitursäure, Amine wie Ethanolamm

Es ist im Allgemeinen vorteilhaft Schritt (ii) in Gegenwart eines Lösungsmittels (Verdünnungsmittel) oder Lösungsmittelgemische durchzuführen. Lösungsmittel werden vorteilhaft in einer solchen Menge eingesetzt, dass das Reaktionsgemisch während der Deallylierung gut rührbar bleibt. Als Lösungsmittel zur Durchführung von Schritt (ii) kommen alle unter den Reaktionsbedingungen inerten organischen Lösungsmittel in Frage, wobei die Art des verwendeten Lösungsmittels von der Art der Deallylierung abhängig ist.

Als Beispiele sind zu nennen Alkohole wie Methanol, Ethanol, Isopropanol, Butanol; Ether, wie Ethylpropylether, Methyl-tert-butylether, n-Butylether, Anisol, Phenetol, Cyclohexylmethylether, Dimethylether, Diethylether, Dimethylglycol, Diphenylether, Dipropylether, Diisopropylether, Di-*n-*butylether, Diisobutylether, Diisoamylether, Ethylenglycoldimethylether, Isopropylethylether, Tetrahydrofuran, Methyl-Tetrahydrofuran, Dioxan, Dichlordiethylether und Polyether des Ethylenoxids und/oder Propylenoxids; Amine wie Trimethyl-, Triethyl-, Tripropyl-, Tributylamin, *N*-Methylmorpholin, Pyridin, alkylierte Pyridine und Tetramethylendiamin; aliphatische, cycloaliphatische oder aromatische Kohlenwasserstoffe wie Pentan, n-Hexan, n-Heptan, n-Oktan, Nonan und technische Kohlenwasserstoffe welche durch Fluor- und Chloratome substituiert sein können, wie Dichlormethan, Trichlormethan, Tetrachlorkohlenstoff, Fluorbenzol, Chlorbenzol oder Dichlorbenzol; Cyclohexan, Methylcyclohexan, Petrolether, Ligroin, Octan, Benzol, Toluol, Brombenzol, Nitrobenzol, Xylol; Ester wie Methyl-, Ethyl-, Butyl-, Isobutylacetat, sowie Dimethyl-, Dibutyl-, Ethylencarbonat; Wasser; organische Säuren wie Ameisensäure, Essigsäure, Trifluoressigsäure, Propionsäure und anorganische Säuren wie Schwefelsäure, Salzsäure, Phosphorsäure.

Von den zuvor genannten Lösungsmitteln sind Wasser, Ethanol und Butanol bevorzugt.

Nach Ende der Deallylierung kann das erhaltene 2,2-Difluorethylamin durch Destillation aufgereinigt werden. Alternativ kann das 2,2-Difluorethylamin auch als Salz, z.B. Hydrochlorid, isoliert und gereinigt werden. Das Salz wird durch Zugabe von Säure vor, während oder nach der Deallylierung erzeugt. Das Salz kann anschließend durch Zugabe von Base wieder freigesetzt werden.

Das 2,2-Difluorethylamin liegt jedoch gewöhnlich in einer solchen Reinheit vor, dass es nach Filtration des Katalysators im Lösungsmittel weiter verwendet werden kann.

Die vorliegende Erfindung wird anhand der nachfolgenden Beispiele näher erläutert, ohne die Erfindung dabei auf diese einzuschränken.

### Herstellungsbeispiele:

### 1. Herstellung von N-(2,2-Difluorethyl)prop-2-en-1-amin (Schritt (i)):

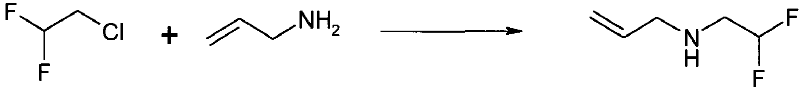

### Beispiel 1.1:

382 g (3,67 mol) 2,2-Difluor-1-chlorethan und 70 g (1,2 mol) Prop-2-en-1-amin werden 16 Stunden bei 120°C in einem Autoklaven erhitzt. Das Reaktionsgemisch wird mit 200 g Wasser versetzt und anschließend werden die Phasen getrennt. Die organische Phase wird bei 55°C destilliert. Man erhält 65 g (entspricht 87,4 % Ausbeute bezogen auf umgesetztes Prop-2-en-1-amin) N-(2,2-Difluorethyl)prop-2-en-1-amin. Unverbrauchtes Prop-2-en-1-amin, das als Hydrochlorid anfällt, kann durch Zugabe von Natronlauge wieder freigesetzt werden.
NMR ¹H (CDCl₃) : 5,76 - 6,0 (m, 2H), 5,22 (m, 1H), 3,31 (m, 2H), 2,96 (dt, 2H)

### Beispiel 1.2:

382 g (3,67 mol) 2,2-Difluor-1-chlorethan und 70 g Prop-2-en-1-amin (1,2 mol) werden 16 Stunden bei 120°C in einem Autoklaven erhitzt. Das Rohgemisch wird anschließend filtriert und der Rückstand mit 150 g 2,2-Difluor-1-chlorethan gewaschen. Die organische Phase wird zuerst bei Normaldruck und 55°C destilliert. Restmengen an 2,2-Difluor-1-chlorethan werden bei 500 mbar entfernt und der Rückstand wird im Vakuum feindestilliert. Man erhält 56 g (entspricht 76 % Ausbeute) N-(2,2-Difluorethyl)prop-2-en-1-amin. Unverbrauchtes Prop-2-en-1-amin, das als Hydrochlorid anfällt, kann durch Zugabe von Natronlauge wieder freigesetzt werden.
NMR ¹H (CDCl₃) : 5,76 - 6,0 (m, 2H), 5,22 (m, 1H), 3,31 (m, 2H), 2,96 (dt, 2H)

### 2. Herstellung von 2,2-Difluorethylamin (Schritt (ii) - Deallylierung):

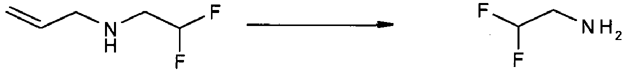

### Beispiel 2.1:

50 g (0,404 mol) N-(2,2-Difluorethyl)prop-2-en-1-amin werden in 253 g (4,1 mol) 2-Aminoethanol gelöst und mit 2,5 g (1,2 mmol) Palladium 10%ig auf Kohle (wasserfeucht) versetzt. Anschließend wird auf 90°C erwärmt. Das entstehende Produkt 2,2-Difluorethylamin wird dann im Vakuum bei 100 mbar und 50°C Innentemperatur abdestilliert. Das Destillat wird erneut feindestilliert. Man erhält 23 g (entspricht 68 % Ausbeute) 2,2-Difluorethylamin.
NMR ¹H (CDCl₃): 5,5 - 5,9 (m, 1H), 2,94 - 3,1 (m, 2 H), 1,26 (br m, NH₂)

## Patentansprüche

1. Ein Verfahren zur Herstellung von 2,2-Difluorethylamin der Formel (I)
CHF₂CH₂NH₂ (I)
umfassend die Schritte (i) und (ii):
Schritt (i): Umsetzung von 2,2-Difluor-1-halogenethan der Formel (II)
CHF₂-CH₂Hal (II)
worin Hal für Chlor, Brom oder Jod steht,
mit Prop-2-en-1-amin der Formel (III) 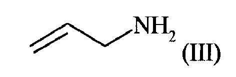 zu N-(2,2-Difluorethyl)prop-2-en-1-amin der Formel (IV) 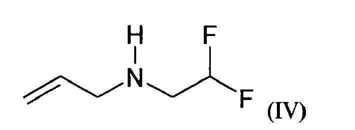 vorzugsweise in Gegenwart eines Säurefängers
und
Schritt (ii): Entfernung der Allylgruppe aus dem in Schritt (i) erhaltenen N-(2,2-Difluorethyl)prop-2-en-1-amin der Formel (IV), wodurch 2,2-Difluorethylamin der Formel (I) oder ein Salz davon erhalten wird.

2. Das Verfahren nach Anspruch 1, wobei Schritt (ii) in Gegenwart eines Katalysators der ein oder mehrere Metalle der Gruppen 8 - 10 des Periodensystems enthält, und gegebenenfalls in Gegenwart eines Nucleophils durchgeführt wird, wobei das Nucleophil ausgewählt ist unter Hydroxiden, Alkoholaten, Thiolaten, Carbanionen, Halogenide, Peroxide, Cyanide und Azide, Thiole, Sulfinsäuren, 2-Mercaptobenzoesäure, Malonsäure und deren Derivate und β-Dicarbonylverbindungen, Barbitursäuren, N,N'-Dimethylbarbitursäure, Amine und Ethanolamm

3. Das Verfahren nach Anspruch 2, wobei der Katalysator ein Palladium-Katalysator ist.

4. Das Verfahren nach Anspruch 3, wobei der Katalysator ausgewählt ist unter Palladium(0)-Katalysatoren, Palladium-10 % ig auf Kohle, Palladium(II)chlorid, Palladium(II)acetat, Bis(acetylacetonat)palladium(II), Dichlorobis(triphenylphosphin)palladium(II), Tetrakis(triethylphosphin)palladium und Tetrakis(triphenylphosphin)palladium.

5. Das Verfahren nach einem der Ansprüche 1 bis 4, wobei ein Teil des verwendeten Prop-2-en-1-amins als Säurefänger dient, während der andere Teil des verwendeten Prop-2-en-1-amins umgesetzt wird.

6. Das Verfahren nach einem der Ansprüche 1 bis 5, wobei im Schritt (i) als Säurefänger eine organische oder anorganische Base eingesetzt wird.

7. Das Verfahren nach einem der Ansprüche 1 bis 6, wobei Schritt (i) ohne Lösungsmittel durchgeführt wird.

8. Das Verfahren nach einem der Ansprüche 1 bis 7, wobei Schritt (i) in Gegenwart eines Katalysators durchgeführt wird, der ausgewählt ist aus Alkalibromiden und -jodiden, Ammoniumbromid, Ammoniumjodid, Tetraalkylammoniumbromide, Tetraalkylammoniumjodide, Tetraalkylphosphoniumhalogenide, Tetraarylphosphoniumhalogenide, Tetrakis(dimethylamino)-phosphoniumbromid, Tetrakis(diethylamino)phosphoniumbromid, Tetrakis(dipropylamino)-phosphoniumchlorid, Tetrakis(dipropylamino)phosphoniumchlorid oder -bromid, Bis(dimethylamino)[(1,3-dimethylimidazolidin-2-yliden)amino]methyliumbromid und Mischungen davon.

9. Das Verfahren nach einem der Ansprüche 1 bis 6, worin in Formel (II) Hal für Chlor steht.

10. Ein Verfahren zur Herstellung von N-(2,2-Difluorethyl)prop-2-en-1-amin umfassend die Umsetzung von 2,2-Difluor-1-chlorethan mit Prop-2-en-1-amin in Gegenwart eines Säurefängers und gegebenenfalls in Gegenwart eines Katalysators.

11. Verwenden von N-(2,2-Difluorethyl)prop-2-en-1-amin zur Herstellung von 2,2-Difluorethylamin, umfassend die Entfernung der Allylgruppe durch Deallylierung.

## Claims

1. A process for the preparation of 2,2-difluoroethylamine of the formula (I)
CHF₂CH₂NH₂ (I)
comprising the stages (i) and (ii):
stage (i): reaction of 2,2-difluoro-1-haloethane of the formula (II)
CHF₂-CH₂Hal (II)
in which Hal is chlorine, bromine or iodine, with prop-2-en-1-amine of the formula (III) 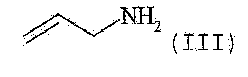 to give N-(2,2-difluoroethyl)prop-2-en-1-amine of the formula (IV) 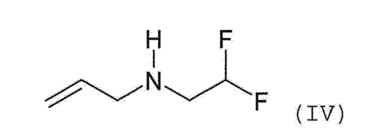 preferably in the presence of an acid scavenger,
and
stage (i-i): removal of the allyl group from the N-(2,2-difluoroethyl)prop-2-en-1-amine of the formula (IV) obtained in stage (i), through which 2,2-difluoroethylamine of the formula (I) or a salt thereof is obtained.

2. The process according to Claim 1, in which stage (ii) is carried out in the presence of a catalyst comprising one or more metals from Groups 8 - 10 of the Periodic Table and optionally in the presence of a nucleophile, the nucleophile being chosen from hydroxides, alkoxides, thiolates, carbanions, halides, peroxides, cyanides and azides, thiols, sulphinic acids, 2-mercaptobenzoic acid, malonic acid and the derivatives thereof, and β-dicarbonyl compounds, barbituric acids, such as, for example, N,N'-dimethylbarbituric acid, amines such as ethanolamine.

3. The process according to Claim 2, in which the catalyst is a palladium catalyst.

4. The process according to Claim 3, in which the catalyst is chosen from palladium(0) catalysts, 10% palladium-on-charcoal, palladium(II) chloride, palladium(II) acetate, bis(acetylacetonate)palladium(II), dichlorobis(triphenylphosphine)palladium(II), tetrakis(triethylphosphine)palladium and tetrakis(triphenylphosphine)palladium.

5. The process according to one of Claims 1 to 4, in which a portion of the prop-2-en-1-amine used acts as acid scavenger, while the other portion of the prop-2-en-1-amine used is reacted.

6. The process according to one of Claims 1 to 5, in which, in stage (i), an organic or inorganic base is used as acid scavenger.

7. The process according to one of Claims 1 to 6, in which stage (i) is carried out without solvent.

8. The process according to one of Claims 1 to 7, in which stage (i) is carried out in the presence of a catalyst chosen from alkali metal bromides and iodizes, ammonium bromide, ammonium iodide, tetraalkylammonium bromides, tetraalkylammonium iodides, tetraalkylphosphonium halides, tetraarylphosphonium halides, tetrakis dimethyl-amino)phosphonium bromide, tetrakis(diethylamino)-phosphonium bromide, tetrakis(dipropylamino)-phosphonium chloride or bromide, bis(dimethylamino)[(1,3-dimethylimidazolidin-2-ylidene)amina]methylium bromide and mixtures thereof.

9. The process according to one of Claims 1 to 6, in which, in formula (II), Hal is chlorine.

10. A process for the preparation of No difluoroethyl)prop-2-en-1-amine, comprising the reaction of 2,2-difluoro-1-chloroethane with prop-2-en-1-amine in the presence of an acid scavenger and optionally in the presence of a catalyst.

11. Use of N-(2,2-difluoroethyl)prop-2-en-1-amine in the preparation of 2,2-difluoroethylamine, comprising the removal of the allyl group by deallylation.

## Revendications

1. Procédé pour la préparation de 2, 2-difluoroéthylamine de formule (I)
CHF₂CH₂NH₂ (I)
comprenant les étapes (i) et (ii) :
Etape (i) transformation de 2,2-difluoro-1-halogénoéthane de formule (II)
CHF₂-CH₂Hal (II)
dans laquelle Hal représente chlore, brome ou iode, avec de la prop-2-én-1-amine de formule (ICI) 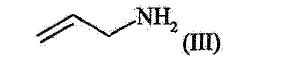 en N-(2,2-difluoroéthyl)prop-2-en-1-amine de formule (IV) 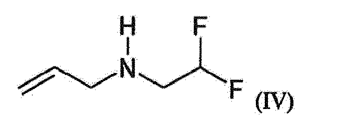 de préférence en présence d'un piège d'acide et
Etape (ii) : élimination du groupe allyle de la N-(2,2-difluoroéthyl)prop-2-én-1-amine de formule (IV) obtenue dans l'étape (i), ce qui permet d'obtenir de la 2,2-difluoroéthylamine de formule (I) ou un sel de celle-ci.

2. Procédé selon la revendication 1, l'étape (ii) étant réalisée en présence d'un catalyseur qui contient un ou plusieurs métaux des groupes 8-10 du système périodique, et le cas échéant en présence d'un nucléophile, le nucléophile étant choisi parmi les hydroxydes, les alcoolates, les thiolates, les carbanions, les halogénures, les peroxydes, les cyanures et les azides, les thiols, les acides sulfiniques, l'acide 2-mercaptobenzoïque, l'acide malonique et leurs dérivés et les composés β-dicarbonyle, les acides barbituriques, l'acide N,N'-diméthylbarbiturique, les amines et l'éthanolamine.

3. Procédé selon la revendication 2, dans lequel le catalyseur est un catalyseur à base de palladium.

4. Procédé selon la revendication 3, le catalyseur étant choisi parmi les catalyseurs du palladium (0), le palladium à 10% sur carbone, le chlorure de palladium (II), l'acétate de palladium (II), le bis(acétylacétonate)-palladium (II), le dichlorobis(triphénylphosphine)-palladium (II), le tétrakis(triéthylphosphine)-palladium et le tétrakis(triphénylphosphine)-palladium.

5. Procédé selon l'une quelconque des revendications 1 à 4, une partie de la prop-2-én-1-amine utilisée servant de piège d'acide, alors que l'autre partie de la prop-2-én-1-amine est transformée.

6. Procédé selon l'une quelconque des revendications 1 à 5, en utilisant dans l'étape (i), comme piège d'acide, une base organique ou inorganique.

7. Procédé selon l'une quelconque des revendications 1 à 6, l'étape (i) étant réalisée sans solvant.

8. Procédé selon l'une quelconque des revendications 1 à 7, 1(étape (i) étant réalisée en présence d'un catalyseur qui est choisi parmi les bromures et les iodures de métal alcalin, le bromure d'ammonium, l'iodure d'ammonium, les bromures de tétraalkylammonium, les iodures de tétraalkylammonium, les halogénures de tétraalkylphosphonium, les halogénures de tétraarylphosphonium, le bromure de tétrakis(diméthylamino)phosphonium, le bromure de tétrakis(diéthylamino)phosphonium, le chlorure de tétrakis(dipropylamino)phosphonium, le chlorure ou le bromure de tétrakis(dipropylamino)phosphonium, le bromure de bis(diméthylamino)[(1,3-diméthylimidazolidin-2-ylidène)amino]méthylium et leurs mélanges.

9. Procédé selon l'une ou plusieurs des revendications 1 à 6, Hal dans la formule (II) représentant chlore.

10. Procédé pour la préparation de N-(2,2-difluoroéthyl)prop-2-én-1-amine comprenant la transformation de 2,2-difluoro-1-chloroéthane avec de la prop-2-én-1-amine en présence d'un piège d'acide et le cas échéant en présence d'un catalyseur.

11. Utilisation de N-(2,2-difluoroéthyl)prop-2-én-1-amine pour la préparation de 2,2-difluoroéthylamine, comprenant l'élimination du groupe allyle par désallylation.
